# EUROPÄISCHE PATENTSCHRIFT

(11) **EP 0 474 037 B1**
(45) Veröffentlichungstag und Bekanntmachung des Hinweises auf die Patenterteilung: **13.07.1994**
(21) Anmeldenummer: 91114026.7
(22) Anmeldetag: 22.08.1991
(51) Int. Cl.: C07D 231/12

(54) **Verfahren zur Herstellung von 3-Methylpyrazol**
Process for the production of 3-methylpyrazole
Procédé pour la production de 3-méthylpyrazole

(30) Priorität: 07.09.1990 DE 4028393
(43) Veröffentlichungstag der Anmeldung: 11.03.1992
(73) Patentinhaber: BASF Aktiengesellschaft, 67063 Ludwigshafen (DE)
(72) Erfinder: Merkle, Hans Rupert, Dr., W-6700 Ludwigshafen (DE); Fretschner, Erich, W-6918 Neckarsteinach (DE)

(56) Entgegenhaltungen:
- EP-A- 0 285 947
- CHEMICAL ABSTRACTS, Band 110, Nr. 9, 27. Februar 1989, Columbus, Ohio, USABERESTEVICH, B.K. "Method of producing 5-amino -3-methylpyrazole" Seite 651,Spalte 2, Zu- sammenfassung-Nr. 75 488p
- CHEMICAL ABSTRACTS, Band 99, Nr. 23, 5. Dezember 1983, Columbus, Ohio, USAKHRIMYAN, A.P. et al. "3(5)-Benzyl-5(3)-methyl- -pyrazole" Seite 754, Spalte 2,Zu- sammenfassung-Nr. 194 953a

## Beschreibung

Die vorliegende Erfindung betrifft ein neues Verfahren zur Herstellung von 3-Methylpyrazol und 5-Methylpyrazol und deren Derivaten der allgemeinen Formel Ia bzw. Ib 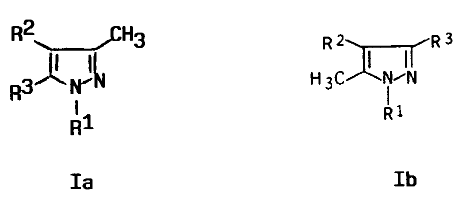
in denen die Reste R¹, R² und R³ für Wasserstoff oder einen C₁-C₈-Alkyl-, C₃-C₈-Cycloalkyl oder einen Aryl- oder Aralkylrest stehen.

In "The Chemistry of Heterocyclic Compounds" Vol. 22, Chapter 3 und 5, werden zahlreiche Synthesewege zum Pyrazol beschrieben, z.B. die Kondensation von α,β-Dicarbonylverbindungen mit Hydrazinen; die Umsetzung von Ethinylcarbonylverbindungen mit Hydrazinen; die Kondensation von Hydrazino-essigester mit 1,2-Diketonen oder die Reaktion in Anwesenheit von ZnCl₂.

Es ist ferner bekannt, 2-Pyrazolin mit Chlor, Alkalimetall- oder Erdalkalimetallhypochloriten (DE-A 30 35 395), mit Schwefel oder Selen (DE-A 30 29 160) oder mit wäßrigem Wasserstoffperoxid (DE-A 34 15 385) zum Pyrazol zu dehydrieren. Weiterhin sind die thermische Gasphasendehydrierung von 2-Pyrazolin an Palladium- oder Platinkontakten (DE-A 32 09 148) sowie die Thermolyse von N-Sulfonyl-2-pyrazolin zum Pyrazol (DE-A 30 35 394) bekannt. In der älteren deutschen Anmeldung P 39 18 979.1 wird die Dehydrierung von 2-Pyrazolin in Schwefelsäure in Gegenwart von Iodverbindungen beschrieben.

Die vorbeschriebenen Verfahren sind jedoch technisch unbefriedigend, sei es, daß sie nur mit mäßigen Ausbeuten verlaufen, daß sie technisch schwer durchführbar sind, daß sie von komplizierten und schwerzugänglichen Einsatzstoffen ausgehen, daß sie die Verwendung von sehr aggressiven Oxidationsmitteln oder teuren Katalysatoren erfordern, daß sie mit der Bildung giftiger Nebenprodukte wie Schwefel- und Selenwasserstoff verbunden sind oder daß sie sich nicht zur Herstellung von N-substituierten Pyrazolen eignen.

Der Erfindung lag daher die Aufgabe zugrunde, 3-Methylpyrazole und 5-Methylpyrazole auf technisch einfachere und wirtschaftlichere Weise zugänglich zu machen.

Entsprechend dieser Aufgabe wurde ein Verfahren zur Herstellung von 3-Methylpyrazol und 5-Methylpyrazol und deren Derivaten der allgemeinen Formel Ia bzw. Ib 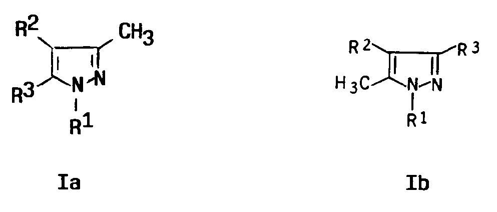
in denen die Reste R¹, R² und R³ für Wasserstoff oder einen C₁-C₈-Alkyl-, C₃-C₈-Cycloalkyl, oder einen Aryl- oder Aralkylrest stehen, wie z.B. Phenyl und Phenyl-C₁-C₄-alkyl, wobei die Phenylreste jeweils auch substituiert sein können, gefunden, das dadurch gekennzeichnet ist, daß man But-2-endiol-(1,4), But-1-endiol-(3,4) oder deren Ester von C₁-C₈-Alkylcarbonsäuren oder aromatischen oder araliphatischen Säuren oder Ethinylalkylcarbinole der Formel II 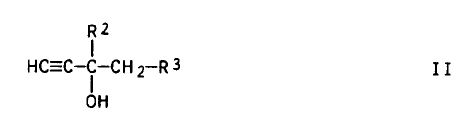
bzw. deren Ester von C₁-C₈-Alkylcarbonsäuren oder aromatischen oder araliphatischen Säuren in 30 bis 100 Gew.-% Schwefelsäure mit ggf. substituiertem Hydrazin der Formel III

R¹-NH-NH₂ III

in der R¹ die obengenannte Bedeutung hat, oder dessen Salzen in Anwesenheit katalytischer Mengen von Iod oder einer Jod oder Jodwasserstoff freisetzenden Verbindung umsetzt.

Bei Verwendung von unsubstituiertem Hydrazin (R¹ = H) sind die Methylpyrazole Ia und Ib identisch.

Die Umsetzung kann für den Fall der Verwendung von Hydrazinhydrat und But-2-endiol-(1,4) sowie Iodwasserstoffsäure als Katalysator durch folgende Reaktionsgleichungen wiedergegeben werden:

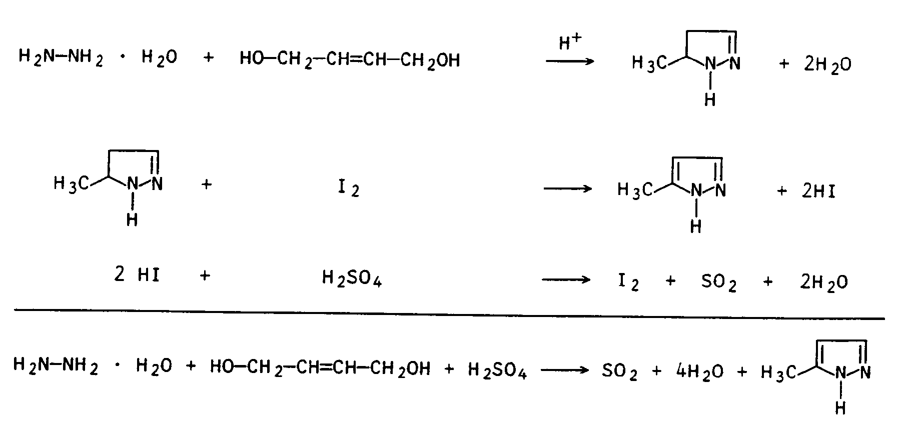

Die Umsetzung von Hydrazinhydrat mit 3-Methylpentin-(1)-ol-(3) kann wie folgt beschreiben werden:

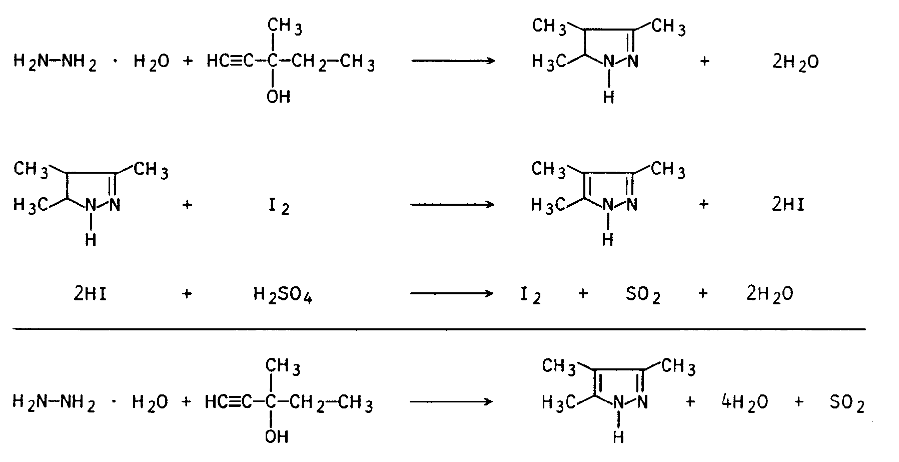

Nach dem erfindungsgemäßen Verfahren ist es vorteilhaft möglich, 3-Methylpyrazole und 5-Methylpyrazole auf technisch einfache Weise im Eintopfverfahren in sehr guten Ausbeuten herzustellen.

Für die Synthese sind beispielsweise folgende Ausgangsstoffe geeignet:
Buten-(2)-diol-(1,4); Buten-(1)-diol-(3,4); Buten-(2)-diol-(1,4)-diacetat; Buten-(2)-diol-(1,4)-dipropionat; Buten-(1)-diol-(3,4)-diacetat; Buten-(1)-diol-(3,4)-dipropionat; Butin-(1)-ol-(3); Pentin-(1)-ol-(3); Hexin-(1)-ol-(3); Heptin-(1)-ol-(3); 3-Methylbutin-(1)-ol-(3); 3-Ethylbutin-(1)-ol-(3); 3-Propylbutin-(1)-ol-(3); 3-Methylpentin-(1)-ol-(3); 3-Ethylpentin-(1)-ol-(3); 3-Propylpentin-(1)-ol-(3); 3-Methylhexin-(1)-ol-(3); 3-Ethylhexin-(1)-ol-(3); 3-Propylhexin-(1)-ol-(3); 3-Methylheptin-(1)-ol-(3); 3-Ethylheptin-(1)-ol-(3); 3-Propylheptin-(1)-ol-(3); 4-Cyclohexylbutin-(1)-ol-(3); 4-Cyclohexyl-3-methylbutin-(1)-ol-(3); 4-Cyclohexyl-pentin-(1)-ol-(3); sowie die entsprechenden Acylate wie z.B. Acetate, Propionate usw. Hierbei spielt die Art der Säurekomponente keine große Rolle. Neben Estern von aliphatischen Carbonsäuren wie C₁-C₈-Alkylcarbonsäuren können auch Ester aromatischer oder araliphatischer Säuren verwendet werden. Die Wahl richtet sich in erster Linie nach Verfügbarkeit, Preis und Abtrennbarkeit aus dem Reaktionsgemisch.

Als zweite Reaktionskomponente sind neben Hydrazin, C₁-C₈-Alkylhydrazine und C₃-C₈-Cycloalkylhydrazine, wie z.B. Methyl-, Ethyl-, Propyl-, Isopropyl-, Butyl-, Isobutyl-, t-Butyl-, Cyclopropyl-, Cyclobutyl-, Cyclopentyl- oder Cyclohexylhydrazin geeignet. Ebenso können Arylhydrazine oder Aralkylhydrazine, wie z.B. Phenylhydrazin und Phenyl-C₁-C₄-alkylhydrazine eingesetzt werden, wobei die Phenylreste jeweils auch substituiert sein können, z.B. durch Halogen wie Fluor, Chlor oder Brom, C₁-C₄-Alkyl oder C₁-C₄-Alkoxy, beispielsweise seien Phenyl- oder Benzylhydrazin genannt.

Anstelle der freien Hydrazinbasen können auch deren Hydrate oder Mineralsäuresalze verwendet werden, wie z.B. die Hydrazinsalze von Schwefelsäure, Salzsäure oder Phosphorsäure.

Als Reste R² und R³ kommen neben Wasserstoff, C₁-C₈-Alkyl insbesondere C₁-C₄-Alkyl sowie die für R¹ im allgemeinen und im einzelnen aufgeführten Reste in Betracht.

Neben elementarem Jod eignen sich als Katalysatoren auch Jodverbindungen wie Jodwasserstoff; Alkalimetall- und Erdalkalimetalliodide wie Lithiumiodid, Natriumiodid, Kaliumiodid, Cäsiumiodid, Magnesiumiodid und Calziumiodid sowie sonstige Metalliodide; es können auch andere anorganische Jodverbindungen wie Erdalkalimetall- und Alkalimetallhypoiodide, -iodite, -iodate -und -periodate oder organische Jodverbindungen wie Alkyliodide, z.B. Methyliodid zur Anwendung kommen.

Schwefelsäure wird als Oxidationsmittel und als Kondensationskatalysator verwendet und kann in verschiedenen Konzentrationen eingesetzt werden. Geeignet sind Konzentrationen zwischen 30 und 100 Gew.-%, bevorzugt zwischen 45 und 90 Gew.-%.

Die Umsetzung wird zweckmäßig so durchgeführt, daß man 1 Mol des entsprechenden Hydrazins mit 0,5 bis 2, insbesondere 0,8 bis 1,5 Mol Butendiolen oder Alkinylalkylcarbinolen bei Temperaturen von 50 bis 250°C, insbesondere 80 bis 200°C, vorzugsweise 110 bis 170°C in Schwefelsäure in Anwesenheit katalytischer Mengen einer Iodverbindung umsetzt, wobei das in der Reaktionsmischung vorhandene und zusätzlich gebildete Wasser teilweise destillativ entfernt werden kann.

Es ist jedoch auch möglich, die Umsetzung bei erhöhtem Druck in geringer konzentrierter Schwefelsäure oder bei entsprechend erhöhter Temperatur bzw. bei verringertem Druck in höher konzentrierter Schwefelsäure oder bei entsprechend niedrigerer Temperatur durchzuführen.

Iod bzw. die Iodverbindung wird bei dieser Umsetzung im allgemeinen in Mengen von 0,01 bis 10 mol-%, insbesondere 0,05 bis 5 mol-% pro Mol Hydrazin eingesetzt.

Die Reaktion kann so durchgeführt werden, daß man alle Reaktionspartner in einem Reaktionsgefäß rührt und auf Reaktionstemperatur bringt, daß man die Reaktanden in ein vorgeheiztes Reaktionsgefäß als Mischung oder getrennt zulaufen läßt, oder daß man einen Teil der Reaktanden bei Reaktionstemperatur vorlegt und die restlichen zutropft.

Schwefelsäure selbst oder Hydrazin-Schwefelsäure können auch im Reaktionsgefäß vorgelegt werden. Unter Destillieren von Wasser wird die Reaktionstemperatur erreicht. Das Einsetzen der Pyrazolbildung erkennt man an der Schwefeldioxidentwicklung. Bei Absorption des Schwefeldioxids mit Natronlauge erhält man äquimolare Mengen Natriumhydrogensulfitlösung von hoher Reinheit.

Beim Abkühlen der Reaktionsmischung kristallisiert das Pyrazol als Schwefelsäuresalz.

Das abdestillierte Wasser enthält den größten Teil des eingesetzten Iodids als Iodwasserstoff, der wieder eingesetzt werden kann. Zur Aufarbeitung neutralisiert man die dunkelbraune Reaktionsmischung, z.B. mit Natronlauge, Ammoniak oder anderen anorganischen Basen und extrahiert sie mehrfach mit einem Lösungsmittel. Nach Trocknung der Extraktionslösung und Eindampfen zur Trockne erhält man die entsprechenden Pyrazole in 85 bis 98 %iger Reinheit, die zur Verbesserung der Reinheit destilliert oder umkristallisiert werden können.

Die neutralisierte Reaktionsmischung kann auch destillativ aufgearbeitet werden, wobei Wasser und reines Pyrazol abdestilliert werden und durch organische Nebenprodukte verunreinigtes Natriumsulfat (oder Ammoniumsulfat) als Destillationsrückstand zurückbleiben. Das bei Neutralisation mit Ammoniak als Destillationssumpf erhaltene verunreinigte Ammoniumsulfat kann oxidativ in Stickstoff und Schwefeldioxid gespalten werden. Letzteres kann wieder über SO₃ in Schwefelsäure übergeführt werden.

Das Verfahren kann kontinuierlich oder diskontinuierlich, drucklos, unter Druck oder schwachem Unterdruck durchgeführt werden.

Die nach dem erfindungsgemäßen Verfahren herstellbaren Pyrazolverbindungen I sind Einsatzstoffe für organische Synthesen z.B. von pharmazeutischen Produkten und Pflanzenschutzmitteln.

### Beispiel 1

Zu einer Lösung von 0,5 g Natriumiodid in 539,0 g 80 %iger Schwefelsäure (4,4 mol) gab man bei 120°C in 90 Minuten eine Mischung aus 100 g (2 mol) Hydrazinhydrat, 193,6 g (2,2 mol) Buten-(2)-diol-(1,4) und 1,5 g Natriumiodid. Nach ungefähr 70 Minuten begann man Wasser abzudestillieren und steigerte allmählich die Reaktionstemperatur auf 155°C.

Dabei wurden 240 g Wasser abdestilliert. Nach Beendigung der Zugabe wurde noch 30 Minuten bei 155°C nachgerührt. Anschließend kühlte man auf 70°C ab, neutralisierte mit 775 g 25 %iger Natronlauge und extrahierte mit 1,2-Dichlorethan.

Die vereinigten Extrakte wurden getrocknet über Natriumsulfat und nach Filtration eingedampft. Man erhielt 155,1 g braunes Öl, woraus durch Destillation im Vakuum 124,3 g farblose Flüssigkeit vom Kp₃₅ 109°C erhalten wurden, die durch Vergleich der physikalisch-chemischen Daten mit denen einer Vergleichsprobe als 3- bzw. 5-Methylpyrazol identifiziert wurde.

### Beispiel 2:

Zu 718,7 g 60 %iger Schwefelsäure (4,4 mol) gab man ein Gemisch von 2 g Natriumiodid in 100 g (2,0 mol) Hydrazinhydrat. Anschließend ließ man bei 90°C in 30 Minuten 378,4 g (2,2 mol) Buten-(1)-diol-(3,4)-di-acetat zutropfen. Durch Abdestillieren von Wasser brachte man die Reaktionstemperatur innerhalb von 3 Stunden auf 140°C. Dann wurde eine weitere halbe Stunde bei 140°C nachgerührt. Insgesamt wurden 680 g Wasser/Essigsäure abdestilliert. Nach Abkühlen auf 70°C wurde mit 1240 g 15 %iger Natronlauge neutralisiert und 4 mal mit 1,2-Dichlorethan extrahiert. Die vereinigten organischen Extrakte wurden über Natriumsulfat getrocknet und nach Filtration eingedampft. Man erhielt 178,3 g eines braunen Öls, aus dem durch Destillation im Vakuum 118 g 3-Methylpyrazol abdestilliert wurden.

### Beispiel 3:

Zu 431,2 g 50 %iger Schwefelsäure (2,2 mol) tropfte man eine Lösung von 1 g Natriumiodid in 50 g (1 mol) Hydrazinhydrat. Zu der sich bildenden Suspension tropfte man innerhalb von 30 Minuten bei 90°C 107,8 g (1,1 mol) 3-Methylpentin-(1)-ol-(3). Durch Abdestillieren von Wasser wurde die Reaktionstemperatur innerhalb von 100 Minuten auf 140°C gesteigert, wobei insgesamt 210 g Wasser abdestillierten. Nach Zugabe eines weiteren Gramms Natriumiodid steigerte man die Reaktionstemperatur auf 150°C und rührte bei dieser Temperatur 15 Minuten nach. Insgesamt wurden 240 g Wasser abdestilliert. Anschließend kühlte man auf 70°C und neutralisierte mit 575 g 15 %iger Natronlauge. Die neutralisierte Reaktionsmischung wurde 4 mal mit 1,2-Dichlorethan extrahiert.

Die vereinigten Extrakte wurden über Natriumsulfat getrocknet und nach Filtration eingedampft. Man erhielt 114,8 g eines braunen Öls, das in 210 g 15 %iger Schwefelsäure gelöst, mit Aktivkohle versetzt und gerührt wurde. Nach Abfiltrieren der Aktivkohle wurde mit 15 %iger Natronlauge alkalisch gestellt, wobei ein Feststoff von Fp. 152°C ausfällt, der durch Elementaranalyse, IR, NMR und Massenspektrum als 3,4,5-Trimethyl-pyrazol identifiziert wurde.

### Beispiel 4:

Zu 308,0 g 70 %iger Schwefelsäure (2,2 mol) gab man 40 g (1 mol) Methylhydrazin und 1 g Natriumiodid. Dazu tropfte man bei 120°C innerhalb von 90 Minuten 96,8 g (1,1 mol) Buten-(2)-diol-(1,4). Durch Abdestillieren von Wasser wurde die Temperatur innerhalb einer halben Stunde auf 130°C gebracht. Bei dieser Temperatur wurde eine weitere halbe Stunde nachgerührt. Insgesamt wurden 155 g Wasser abdestilliert. Anschließend wurde die Reaktionsmischung mit 655 g 15 %iger Natronlauge bei 70°C neutralisiert und mit 1,2-Dichlorethan extrahiert. Die vereinigten organischen Auszüge wurden getrocknet, filtriert und eingedampft. Man erhielt 58,5 g eines braunroten Öls.

Durch Destillation im Vakuum erhielt man in 51 %iger Ausbeute eine Flüssigkeit von Kp₉₀ 81°C, die durch IR- und NMR-Spektren als ein 1:1 Gemisch von 1,3- und 1,5-Dimethylpyrazol identifiziert wurde.

### Beispiel 5

Zu 431,2 g 50 %iger Schwefelsäure (2,2 mol) gab man 46 g (1 mol) Methylhydrazin und 1 g Natriumiodid. Innerhalb von 30 Minuten ließ man bei 90°C 107,8 g (1,1 mol) 3-Methylpentin-(1)-ol-(3) zutropfen und heizte innerhalb von 105 Minuten auf 155°C. Während dieser Dauer und der 30 minütigen Nachrührzeit wurden insgesamt 280 g Wasser abdestilliert. Anschließend verdünnte man die Reaktionslösung wieder mit 500 g Wasser und neutralisierte bei 70°C mit 128,7 g 50 %iger Natronlauge. Man extrahierte dreimal mit 1,2-Dichlorethan und trocknete die vereinigten organischen Auszüge über Natriumsulfat. Nach Filtration und Eindampfen erhielt man ein braunes Öl, das im Vakuum destilliert wurde. Man erhielt 101,8 g farbloses Öl (82,1 % d. Th.) vom Kp₆₃ 104°C, das durch IR- und NMR-Spektren als 1,3,4,5-Tetramethylpyrazol identifiziert wurde.

## Patentansprüche

1. Verfahren zur Herstellung von 3- und 5-Methylpyrazol und deren Derivaten der allgemeinen Formeln Ia bzw. Ib 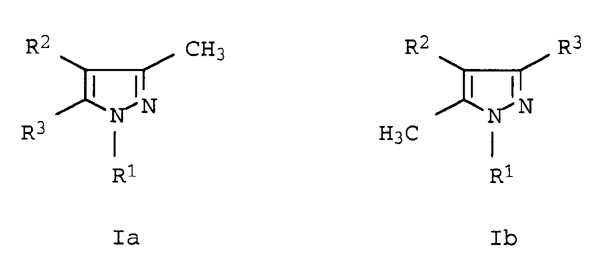 in denen die Reste R¹, R² und R³ für Wasserstoff oder einen C₁-C₈-Alkyl-, C₃-C₈-Cycloalkyl-, oder einen Aryl- oder Aralkylrest wie z.B. Phenyl und Phenyl-C₁-C₄-alkyl, wobei die Phenyleste jeweils auch substituiert sein können, stehen, dadurch gekennzeichnet, daß man But-2-endiol-(1,4), But-1-en-diol-(3,4) oder deren Ester von C₁-C₈-Alkylcarbonsäuren oder aromatischen oder araliphatischen Sauren oder Ethinylalkylcarbinole der Formel II 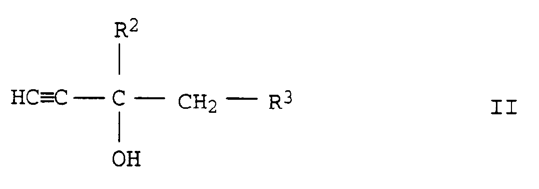 bzw. deren Ester von C₁-C₈-Alkylcarbonsäuren oder aromatischen oder araliphatischen Säuren in 30 bis 100 Gew.-% Schwefelsäure mit ggf. substituiertem Hydrazin der Formel III
R¹-NH-NH₂ III
in der R¹ die obengenannte Bedeutung hat, oder dessen Salzen in Anwesenheit katalytischer Mengen von Jod oder einer Jod oder Jodwasserstoff freisetzenden Verbindung umsetzt.

2. Verfahren zur Herstellung von 3-Methylpyrazol und dessen Derivaten der allgemeinen Formel Ia nach Anspruch 1, in der R¹ Wasserstoff bedeutet, dadurch gekennzeichnet, daß man Hydrazin [H₂N-NH₂] oder dessen Salze verwendet.

3. Verfahren nach den Ansprüchen 1 und 2, dadurch gekennzeichnet, daß man die Umsetzung bei Temperaturen von 50 bis 250°C vornimmt.

4. Verfahren nach den Ansprüchen 1, 2 und 3, dadurch gekennzeichnet, daß man die Umsetzung in Gegenwart von 0,01 bis 10 mol-%, bezogen auf das Hydrazin III, an Jod oder einer Jod oder Jodwasserstoff freisetzenden Verbindung vornimmt.

## Claims

1. A process for preparing 3- and 5-methylpyrazole and their derivatives of the formulae Ia and Ib 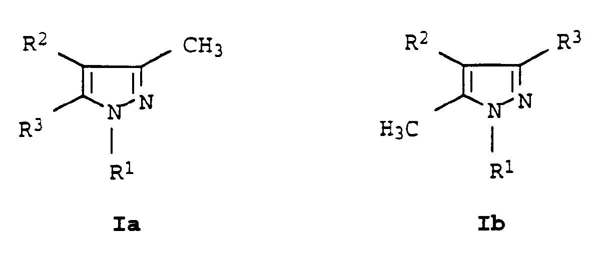 where R¹, R² and R³ are each hydrogen or C₁-C₈-alkyl, C₃-C₈-cycloalkyl, or aryl or aralkyl such as phenyl and phenyl-C₁-C₄-alkyl, where each phenyl can also be substituted, which comprises reacting 2-butene-1,4-diol, 1-butene-3,4-diol or their esters of C₁-C₈-alkylcarboxylic acids or aromatic or araliphatic acids or ethynylalkylcarbinols of the formula II 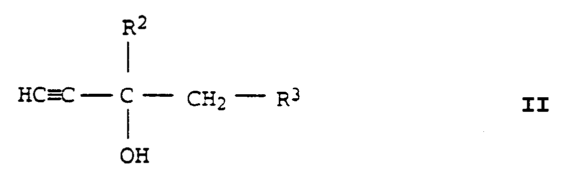 or their esters of C₁-C₈-alkylcarboxylic acids or aromatic or araliphatic acids in 30-100% by weight sulfuric acid with unsubstituted or substituted hydrazine of the formula III
R¹-NH-NH₂ III
where R¹ has the abovementioned meaning, or its salts, in the presence of catalytic amounts of iodine or a compound which liberates iodine or hydrogen iodide.

2. A process for preparing 3-methylpyrazole and its derivatives of the formula Ia as claimed in claim 1, where R¹ is hydrogen, which comprises using hydrazine [H₂N-NH₂] or its salts.

3. A process as claimed in claims 1 and 2, wherein the reaction is carried out at from 50 to 250°C.

4. A process as claimed in any of claims 1, 2 and 3, wherein the reaction is carried out in the presence of from 0.01 to 10 mol % iodine, or a compound which liberates iodine or hydrogen iodide, based on the hydrazine III.

## Revendications

1. Procédé de préparation de 3- et 5-méthylpyrazole et ses dérivés de la formule générale Ia et Ib 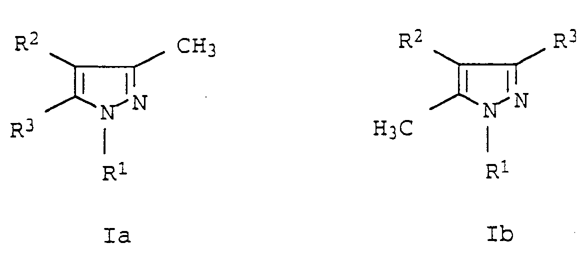 dans lesquelles les restes R¹, R² et R³ sont mis pour hydrogène ou un reste alkyle en C₁-C₈, cycloalkyle en C₃-C₈, ou un reste aryle ou aralkyle, tel que phényle et phényle alkyle en C₁-C₄, les restes phényle pouvant chacun être aussi substitués,
caractérisé par le fait qu'on fait réagir du bu-2-endiol-(1,4), but-1-endiol-(3,4) ou leurs esters d'acides (alkyle en C₁-C₈)-carboxyliques ou d'acides aromatiques ou araliphatiques ou d'éthinylalkylcarbinoles de formule II 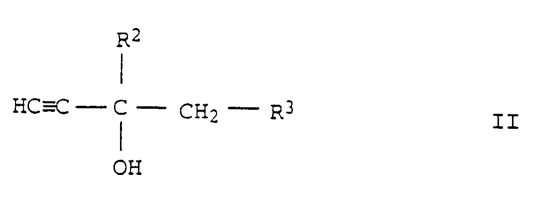 ou leurs esters d'acides (alkyle en C₁-C₈)-carboxyliques ou acides aromatiques ou araliphatiques dans 30 à 100 % en poids d'acide sulfurique avec de l'hydrazine éventuellement substituée de formule III
R¹-NH-NH₂ III
dans laquelle R¹ a la signification sus-indiquée, ou ses sels en présence de quantités catalytiques d'iode ou d'un composé libérant de l'iode ou de l'hydrate d'iode.

2. Procédé de préparation de 3-méthylpyrazole et ses dérivés de la formule générale Ia selon la revendication 1, dans laquelle R¹ représente hydrogène, caractérisé par le fait que l'on utilise l'hydrazine (H₂N-NH₂) ou ses sels.

3. Procédé selon la revendication 1 ou 2, caractérisé par le fait que l'on effectue la réaction à des températures de 50 à 250°C.

4. Procédé selon les revendications 1, 2 et 3, caractérisé par le fait que l'on effectue la réaction en présence de 0,01 à 10 mol %, rapporté à l'hydrazine III, d'iode ou d'un composé libérant de l'iode ou de l'hydrate d'iode.
